# EUROPEAN PATENT SPECIFICATION

(11) **EP 3 053 505 B1**
(45) Date of publication and mention of the grant of the patent: **04.01.2023**
(21) Application number: 13894802.1
(22) Date of filing: 30.09.2013
(51) Int. Cl.: A61B 1/00, A61B 5/00, A61B 5/107

(54) **IMAGE DIAGNOSTIC DEVICE AND GUIDE WIRE ACCESS PATH DETERMINATION SUPPORT**
BILDDIAGNOSEVORRICHTUNG UND UNTERSTÜTZUNG DER BESTIMMUNG EINES FÜHRUNGSDRAHTZUGANGSWEGS
DISPOSITIF DE DIAGNOSTIC D'IMAGE ET SUPPORT DE DÉTERMINATION DE VOIE D'ACCÈS DE FIL-GUIDE

(43) Date of publication of application: 10.08.2016
(73) Proprietor: Terumo Kabushiki Kaisha, Tokyo 151-0072 (JP)
(72) Inventor: ONIMURA, Yuuji, Fujinomiya-shi Shizuoka 418-0015 (JP)
(74) Representative: KIPA AB
(86) International application number: PCT/JP2013/005815
(87) International publication number: WO 2015/044987

(56) References cited:
- WO-A1-2012/132211
- WO-A1-2013/042231
- JP-A- 2006 020 944
- JP-A- 2012 505 669
- US-A1- 2010 174 196
- WANG ANCONG ET AL: "3D assessment of stent cell size and side branch access in intravascular optical coherence tomographic pullback runs", COMPUTERIZED MEDICAL IMAGING AND GRAPHICS, vol. 38, no. 2, 7 September 2013 (2013-09-07), pages 113-122, XP028608242, ISSN: 0895-6111, DOI: 10.1016/J.COMPMEDIMAG.2013.08.007
- CARLO DI MARIO ET AL: "Optical coherence tomography for guidance in bifurcation lesion treatment", EUROINTERVENTION, vol. 6, no. J, 1 December 2010 (2010-12-01), pages J99-J106, XP055364249, FR ISSN: 1774-024X, DOI: 10.4244/EIJV6SUPJA16

## Description

### Technical Field

The present invention relates to an imaging apparatus for diagnosis which uses optical interference and a support method of determining an access route for a guide wire.

### Background Art

Endovascular therapy is performed by using a high-functioning catheter such as a balloon catheter, a stent, or the like. In order to perform preoperative diagnosis or to verify postoperative results, an imaging apparatus for diagnosis such as an optical coherence tomography (OCT) apparatus or the like has been generally used.

The optical coherence tomography apparatus includes a built-in optical fiber, to a distal end of which an imaging core having an optical lens and an optical mirror is attached, and applies a probe, at least a distal end portion of which has a transparent sheath. Then, the probe is inserted into a blood vessel of a patient. While the imaging core is rotated, light is emitted to a vascular wall via the optical mirror. The reflection light from the vascular wall is received again via the optical mirror, and radial scanning is performed, thereby configuring a cross-sectional image of the blood vessel, based on the obtained reflection light. Then, while the optical fiber is rotated, a pulling operation (generally called pullback) is performed at a predetermined speed, thereby forming a three-dimensional image of a lumen surface in the longitudinal direction of the blood vessel (refer to PTL 1). In addition, as an improved type of OCT, a swept-source optical coherence tomography (SS-OCT) apparatus has also been developed.

### Citation List

### Patent Literature

[PTL 1] JP-A-2007-267867

WANG ANCONG ET AL: "3D assessment of stent cell size and side branch access in intravascular optical coherence tomographic pullback runs", COMPUTERIZED MEDICAL IMAGING AND GRAPHICS, vol. 38, no. 2, 7 September 2013 (2013-09-07), pages 113-122, XP028608242, ISSN: 0895-6111, DOI: 10.1016/J.COMPMEDIMAG.2013.08.007, discloses a side branch lumen (gray area) projected onto a cylinder and opened to show its relative location with respect to the stent cells; gray value inside a contour as the distance transform result, where MCUSA (Maximum Circular Unsupported Surface Area) is detected by searching for the highest intensity pixel in it; that linear extrusion of the contour generates the contour projection to segment the access area on the side branch lumen surface (blue); and an example of a side branch access through the stent cell from the original phantom data set, where the brightest region in the image is the side branch access to the main vessel through this stent cell.

CARLO DI MARIO ET AL: "Optical coherence tomography for guidance in bifurcation lesion treatment", EUROINTERVENTION, vol. 6, no. J, 1 December 2010 (2010-12-01), pages J99-J106, XP055364249, ISSN: 1774-024X, DOI: 10.4244/EIJV6SUPJA16, discloses that optical coherence tomography (OCT) provides high quality images of the lumen-intima interface thereby allowing automatic lumen area measurements that can be used to characterise stenosis severity.

### Summary of Invention

### Technical Problem

In general, many bifurcated portions are observed in a blood vessel. In many cases, manual skills are used in order to cause a stent to indwell these bifurcated portions in the blood vessel. In a technique of causing the stent to indwell the bifurcated portions, secondary treatment is sometimes performed on the stent which indwells a main duct. For example, in order to secure blood flow from the main duct to a laterally bifurcated portion in the blood vessel, manual skills for expanding a cell located at an entrance of the laterally bifurcated portion in the stent indwelling the main duct, or manual skills for causing another stent to enter and indwell the laterally bifurcated portion through the cell of the stent indwelling the main duct are used.

Both the manual skills allow a guide wire to enter the laterally bifurcated portion through one cell of the stent indwelling the main duct. However, if the wrong cell is selected and the cell is deformed by the guide wire, there is a possibility that an undesirable situation may happen, for example, that a stent strut may come into poor contact with a vascular wall. Furthermore, if the cell of the stent is deformed once, the cell is less likely to be restored. Therefore, it is important to select the cell through which the guide wire is caused to pass in the above-described manual skills. However, in actual practice, which cell should be selected depends on an operator's experience or capability.

The present invention is made in view of the above-described problems. Then, according to the present specification, there are provided an imaging apparatus for diagnosis and a control method thereof in order to present an operator with guidelines for selecting which portion of a stent indwelling a bifurcated portion of a blood vessel is desirable to allow a guide wire to pass.

### Solution to Problem

In order to solve the above-described problems, an imaging apparatus for diagnosis according to independent claim 1, and a computer program according to independent claim 8 are provided.

### Advantageous Effects of Invention

According to the present invention, it is possible to present an operator with guidelines for selecting which portion of a stent indwelling a bifurcated portion of a blood vessel is desirable to allow a guide wire to pass.

Other features and advantages of the present invention will become apparent from the following description made with reference to the accompanying drawings. In the accompanying drawings, the same reference numerals are given to the same or similar configuring elements.

### Brief Description of Drawings

The accompanying drawings are included in the specification, configure a part of the specification, illustrate embodiments of the present invention, and are used so as to describe principles of the present invention together with the description.
Fig. 1 is a view illustrating an example of an overall configuration of an imaging apparatus for diagnosis 100 according to an embodiment of the present invention.
Fig. 2 is a block configuration diagram of the imaging apparatus for diagnosis 100 according to a first embodiment.
Fig. 3A is a view for describing radial scanning inside a blood vessel.
Fig. 3B is a view for describing a malapposition.
Fig. 4 is a view illustrating a relationship between a two-dimensional vascular cross-sectional image and a three-dimensional vascular lumen surface image.
Fig. 5 is a view illustrating a display example of a two-dimensional vascular lumen image.
Fig. 6 is a block diagram illustrating a functional configuration example for realizing cell selection support according to an embodiment.
Fig. 7A is a view for describing area measurement of a cell.
Fig. 7B is a view for describing the area measurement of the cell.
Fig. 8 is a view for describing a point depending on a position of the cell.
Fig. 9 is a view for describing a region for measuring the malapposition.
Fig. 10 is a view for describing angle measurement of a stent strut.
Fig. 11A is a flowchart for describing processes of the cell selection support.
Fig. 11B is a flowchart for describing processes of the cell selection support.

### Description of Embodiments

Hereinafter, embodiments according to the present invention will be described in detail with reference to the accompanying drawings.

### First Embodiment

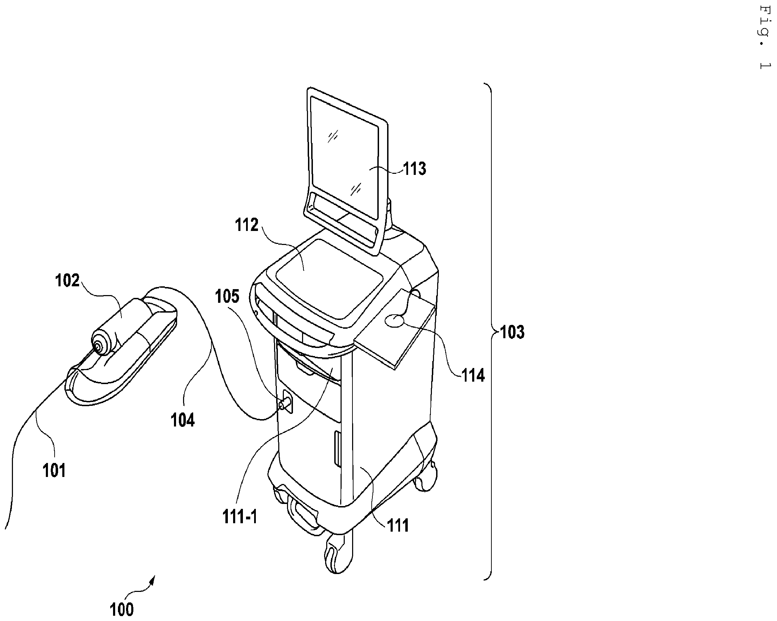

Fig. 1 is a view illustrating an example of an overall configuration of an imaging apparatus for diagnosis 100 which utilizes a swept source according to an embodiment of the present invention.

The imaging apparatus for diagnosis 100 is configured to include a probe 101, a pullback unit 102, and an operation control device 103. The pullback unit 102 and the operation control device 103 are connected to each other by using a cable 104 via a connector 105. The cable 104 accommodates an optical fiber and various signal lines.

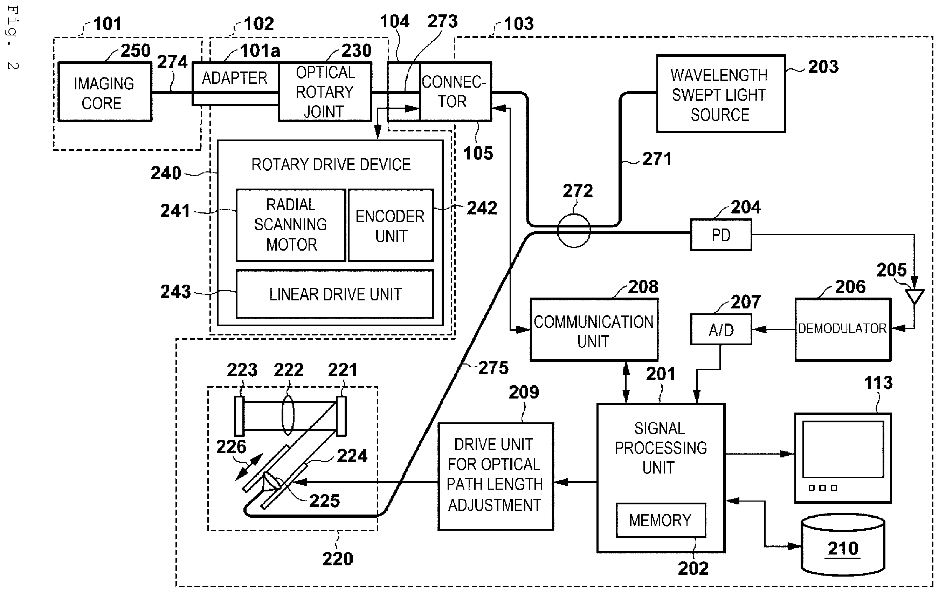

The probe 101 accommodates the optical fiber so as to be rotatable. A distal end of the optical fiber is provided with an imaging core 250 (refer to Fig. 2) having an optical transceiver which transmits light (measurement light), transmitted from the operation control device 103 via the pullback unit 102, in a direction substantially perpendicular to a central axis of the optical fiber, and which receives reflected light of the transmitted light from the outside.

The pullback unit 102 holds the optical fiber accommodated inside the probe 101 via an adapter disposed in the probe 101. Then, the optical fiber accommodated inside the probe 101 is rotated by driving a motor incorporated in the pullback unit 102. In this manner, the imaging core disposed in the distal end of the optical fiber can be rotated. In addition, the pullback unit 102 drives a motor disposed in an incorporated linear drive unit, and performs a process of pulling back the optical fiber accommodated inside the probe 101 at a predetermined speed (reason that the pullback unit is called).

According to the above-described configuration, the probe is guided into a patient's blood vessel, a radial scanning motor 241 (refer to Fig. 2) incorporated in the pullback unit 102 is driven, and the optical fiber accommodated inside the probe is rotated, thereby enabling a vascular wall and a lumen surface to be fully scanned at an angle of 360 degrees. Furthermore, the pullback unit 102 causes the linear drive unit (refer to the reference numeral 243 in Fig. 2) to pull back the optical fiber accommodated inside the probe 101 at a predetermined speed so as to perform scanning along a vascular axis. As a result, it is possible to construct a tomographic image viewed from the inside of the blood vessel.

The operation control device 103 has a function to comprehensively control operations of the imaging apparatus for diagnosis 100. For example, the operation control device 103 comprises a function to input various setting values based on a user's instruction to the apparatus, and a function to process data obtained by measurement and to display the data as a tomographic image of a body lumen.

The operation control device 103 has a main body control unit 111, a printer/DVD recorder 111-1, an operation panel 112, an LCD monitor 113, and the like. The main body control unit 111 generates an optical tomographic image. The optical tomographic image is generated in such a way that interference light data is generated by causing reflected light obtained by measurement to interfere with reference light obtained by splitting light from a light source, and the line data generated based on the interference light data is processed.

The printer/DVD recorder 111-1 prints a process result in the main body control unit 111, or stores the process result as data. The operation panel 112 is a user interface to which a user inputs various setting values or instructions. The LCD monitor 113 functions as a display device, and displays a tomographic image generated in the main body control unit 111, for example. The reference numeral 114 represents a mouse serving as a pointing device (coordinate input device).

Next, a functional configuration of the imaging apparatus for diagnosis 100 will be described. Fig. 2 is a block configuration diagram of the imaging apparatus for diagnosis 100. Hereinafter, the functional configuration of swept source OCT will be described with reference to the drawing.

In the illustration, the reference numeral 201 represents a signal processing unit which controls the overall imaging apparatus for diagnosis, which is configured to include a microprocessor and several circuits. The reference numeral 210 is a non-volatile storage device represented by a hard disk which stores various programs or data files executed by the signal processing unit 201. Furthermore, a stent database (details to be described later) is stored therein. The reference numeral 202 represents a memory (RAM) disposed inside the signal processing unit 201. The reference numeral 203 is a wavelength swept light source which is a light source for repeatedly generating light having a wavelength varying within a preset range along the time axis.

The light output from the wavelength swept light source 203 is incident on one end of a first single mode fiber 271, and is transmitted toward a distal side thereof. The first single mode fiber 271 is optically coupled to a fourth single mode fiber 275 in an optical fiber coupler 272 located in the middle therebetween.

The light emitted from the distal side by the optical fiber coupler 272 in the first single mode fiber 271 is guided to a second single mode fiber 273 via the connector 105. The other end of the second single mode fiber 273 is connected to an optical rotary joint 230 accommodated inside the pullback unit 102.

On the other hand, the probe 101 has an adapter 101a for connecting the pullback unit 102 thereto. Then, the probe 101 is connected to the pullback unit 102 by the adapter 101a, thereby allowing the probe 101 to be stably held in the pullback unit 102. Furthermore, an end portion of a third single mode fiber 274 rotatably accommodated inside the probe 101 is connected to the optical rotary joint 230. As a result, the second single mode fiber 273 and the third single mode fiber 274 are optically coupled to each other. The imaging core 250 equipped with a mirror and a lens which emit the light in a direction substantially perpendicular to the rotation axis is disposed in the other end (leading side of the probe 101) of the third single mode fiber 274.

As a result of the above-described configuration, the light emitted from the wavelength swept light source 203 is guided to the imaging core 250 disposed in the end portion of the third single mode fiber 274 via the first single mode fiber 271, the second single mode fiber 273, and the third single mode fiber 274. The imaging core 250 emits the light in a direction perpendicular to the fiber axis. The received reflected light is reversely guided in turn, and is caused to return to the operation control device 103.

On the other hand, an optical path length adjustment mechanism 220 which finely adjusts an optical path length of the reference light is disposed in an end portion opposite to the fourth single mode fiber 275 coupled to the optical fiber coupler 272. The optical path length adjustment mechanism 220 functions as optical path length changing means for changing the optical path length corresponding to length variations thereof so that the length variations of the individual probe units 101 can be absorbed, for example, in a case where the probe unit 101 is replaced. Therefore, a collimating lens 225 located in the end portion of the fourth single mode fiber 275 is disposed on a one-axis stage 224 which is movable in an optical axis direction thereof as illustrated by an arrow 226.

Specifically, the one-axis stage 224 functions as the optical path length changing means having enough of a variable range of the optical path length to absorb the variations in the optical path length of the probe unit 101 in a case where the probe unit 101 is replaced. Furthermore, the one-axis stage 224 also comprises a function as adjusting means for adjusting an offset. For example, even in a case where the distal end of the probe unit 101 is not in close contact with a surface of body tissues, the one-axis stage finely changes the optical path length. In this manner, it is possible to set the optical path length in a state of interfering with the reflected light from a surface position of the body tissues.

The optical path length is finely adjusted by the one-axis stage 224. The light reflected on a mirror 223 via a grating 221 and a lens 222 is guided again to the fourth single mode fiber 275, is mixed with the light obtained from the first single mode fiber 271 side by the optical fiber coupler 272, and then is received by a photodiode 204 as interference light.

The interference light received by the photodiode 204 in this way is photoelectrically converted, and is input to a demodulator 206 after being amplified by an amplifier 205. The demodulator 206 performs demodulation processing for extracting only a signal portion of the interference light, and an output therefrom is input to an A/D converter 207 as an interference light signal.

The A/D converter 207 performs sampling on an interference light signal, for example, at 90 MHz by the amount of 2,048 points, and generates digital data (interference light data) of one line. The reason for setting the sampling frequency to 90 MHz is on the assumption that approximately 90% of swept source cycles (25 µsec) is extracted as the digital data of 2,048 points, in a case where a repetition frequency of the swept source is set to 40 kHz. However, the reason is not particularly limited thereto.

The interference light data in the units of lines which is generated by the A/D converter 207 is input to the signal processing unit 201, and is temporarily stored in the memory 202. Then, the signal processing unit 201 generates data in a depth direction (line data) by performing frequency resolution on the interference light data using the fast Fourier transform (FFT), and the data is subjected to coordinate transformation. In this manner, an optical cross-sectional image is constructed at each intravascular position, and is output to the LCD monitor 113 at a predetermined frame rate.

The signal processing unit 201 is further connected to a drive unit for optical path length adjustment 209 and a communication unit 208. The signal processing unit 201 controls a position of the one-axis stage 224 (optical path length control) via the drive unit for optical path length adjustment 209.

The communication unit 208 has several types of drive circuits incorporated therein, and communicates with a rotary drive device 240 accommodated inside the pullback unit 102 under the control of the signal processing unit 201. Specifically, the communication unit 208 supplies a drive signal to the radial scanning motor 241 in order to cause the optical rotary joint 230 inside the pullback unit 102 to rotate the third single mode fiber 274, receives a signal from an encoder unit 242 in order to detect a rotation position of the radial motor 241, and supplies a drive signal to a linear drive unit 243 in order to pull back the third single mode fiber 274 at a predetermined speed.

The above-described process in the signal processing unit 201 is also realized by a predetermined program causing a computer to execute the process.

In the above-described configuration, a user locates the probe 101 at a diagnosis-targeted vascular position (coronary artery or the like) of a patient, and the user operates the probe 101 so as to discharge a transparent flush solution(usually, a physiological salt solution or a contrast agent) into the blood vessel through the probe distal end. The reason is to exclude the influence of the blood during the imaging. Then, if the user inputs an instruction to start scanning, the signal processing unit 201 drives the wavelength swept light source 203, and drives the radial scanning motor 241 and the linear drive unit 243 (hereinafter, a light emitting and light receiving process performed by driving the radial scanning motor 241 and the linear drive unit 243 will be referred to as scanning). As a result, swept source light from the wavelength swept light source 203 is supplied to the imaging core 250 through the above-described route. At this time, the imaging core 250 located at the distal position of the probe 101 moves along the rotation axis while rotating. Accordingly, the imaging core 250 emits the light onto a vascular lumen surface, and receives the reflected light while rotating and moving along the vascular axis.

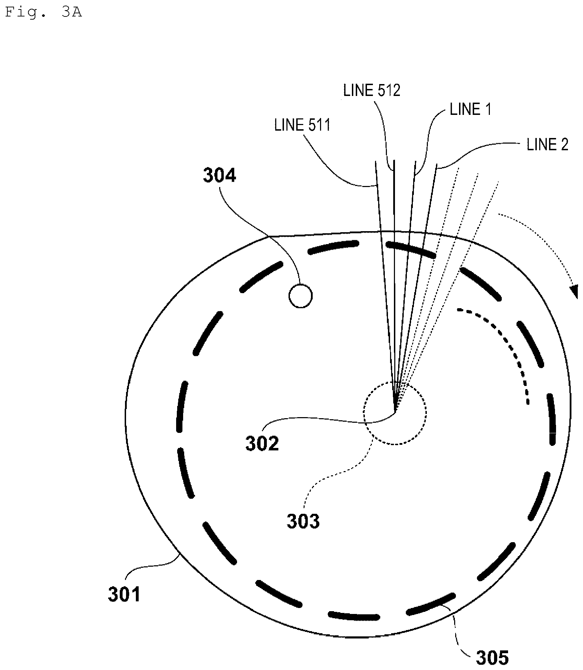

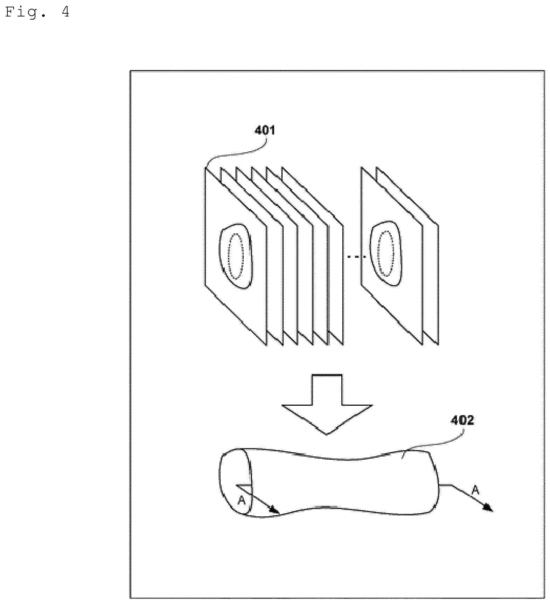

Here, a process for generating one optical cross-sectional image will be briefly described with reference to Fig. 3A. Fig. 3A is a view for describing a reconfiguration process of a cross-sectional image of a vascular lumen surface 301 on which the imaging core 250 is located. While the imaging core 250 is rotated once (360 degrees), measurement light is transmitted and received multiple times. The light transmitted and received once can obtain data of one line in a light-emitted direction. Therefore, for example, if the light is transmitted and received 512 times during one rotation, it is possible to obtain 512 line data items radially extending from a rotation center 302. The 512 line data items become dense in the vicinity of the rotation center position, and become isolated from each other as the line data items are located further away from the rotation center position. Therefore, a pixel in an empty space between the respective lines is generated by performing known interpolation processing, thereby generating two-dimensional cross-sectional images which are visible to humans. Then, as illustrated in Fig. 4, the generated two-dimensional cross-sectional images 401 are connected to each other along the vascular axis. In this manner, it is possible to obtain a three-dimensional vascular image 402. It should be noted that the center position of the two-dimensional cross-sectional image coincides with the rotation center position of the imaging core 250, but is not the center position of the vascular cross section.

According to the imaging apparatus for diagnosis which utilizes a swept source, during a period while the light corresponding to one certain line in Fig. 3A is output and received, the wavelength swept light source 203 gradually changes a wavelength of the light output to the time axis. The wavelength swept light source 203 has a known configuration, and thus, will not be particularly described. The wavelength swept light source 203 outputs the light having a wavelength from λmax to λmin during a period while the light corresponding to one line is output and received. In other words, the period of λmax:λmin is a period (in the embodiment, 25 µsec) needed to obtain data corresponding to one line in Fig. 3A.

When the light is transmitted and received, the light is also reflected from a catheter sheath itself of the probe 101. Accordingly, as illustrated, a shadow 303 of the catheter sheath is formed on the cross-sectional image. In addition, the reference numeral 304 in the drawing represents a shadow of a guide wire which guides the probe 101 to a lesion area. Then, the reference numeral 305 is a shadow of a stent. The guide wire or the stent is made of metal, and the light is not transmitted therethrough. Accordingly, it is not possible to obtain an image of a rear side portion of the guide wire or the stent when viewed from the rotation center 302. It should be recognized that the illustration is only a conceptual diagram.

In the present embodiment, a bifurcated portion of the stent and the blood vessel is identified from information obtained by performing pullback scanning. Then, a route for the guide wire (auxiliary information for selecting a cell through which the guide wire is to pass) is displayed, based on a relationship between a cell formed by the identified stent and the bifurcated portion.

For this reason, it is necessary to extract information for identifying the stent from the information obtained by performing the pullback scanning. An example thereof will be described below.

First, as a method of identifying the stent from an image, since the stent portion is made of metal, the stent is extremely bright on the image, compared to vascular tissues. Therefore, a threshold value which enables the stent to be identified may be set so that pixels having the threshold value or greater are regarded as pixels configuring the stent. However, the guide wire (reference numeral 304 in Fig. 3A) is also made of metal, and the guide wire is also very bright. Accordingly, the brightness alone does not enable the guide wire to be identified. However, as is understood from Fig. 3A, when bright portions are connected to each other, a shape thereof forms a gentle circle or oval. That is, based on the gentle circle or oval, very bright pixels beyond a predetermined threshold value are regarded as the guide wire. If the pixels are excluded, only the stent can be identified.

Next, a process of extracting the stent will be described. Based on the information obtained by performing the pullback scanning, a two-dimensional planar development image obtained by cutting out the stent using a sectional image taken along line A-A in fig. 4 is calculated. However, as illustrated in Fig. 3A, the rotation center position 302 of the imaging core 250 is not necessarily located at the center of the stent. That is, the stent has a portion which is far from the rotation center position 302 and a portion which is close therefrom. As is well known, if an object is closer to a viewpoint position, the object looks bigger. If the object is farther therefrom, the object looks smaller. Therefore, in a case where the stent is simply cut out using the sectional view taken along line A-A in Fig. 4, a portion of the stent which is close to the rotation center position 302 is enlarged in appearance, and a portion of the stent which is far therefrom is reduced in appearance. Fortunately, the distance from the rotation center position 302 to the portion of the stent can be calculated using scanning data. According to the embodiment, a process of correcting the enlargement and reduction is performed in accordance with the distance, thereby generating a two-dimensional planar image obtained by cutting the stent using the sectional view taken along line A-A in fig. 4 so that the portions of the stent over the entire periphery are regarded as equally away from the rotation center position 302. Hereinafter, the two-dimensional planar image is referred to as a two-dimensional vascular lumen image.

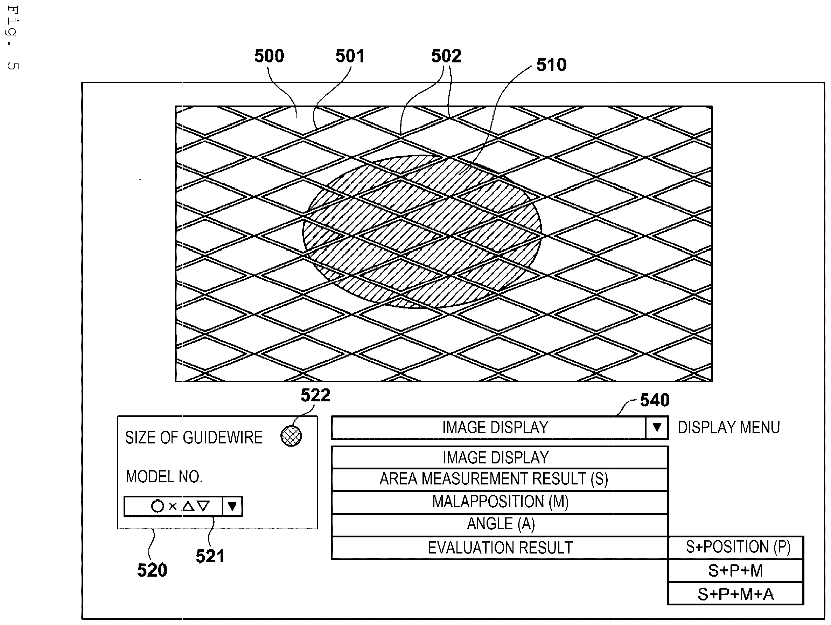

If the above-described two-dimensional vascular lumen image is generated, an image of the stent in the two-dimensional vascular lumen image is uniformly distributed without depending on the position of the stent, thereby forming an aligned image. Accordingly, it is possible to very accurately extract characteristic information of the stent. If the above-described two-dimensional vascular lumen image is generated, the image of the stent in the two-dimensional vascular lumen image does not depend on the position of the stent, and forms an image based on the actual size. Fig. 5 illustrates a state where the image of the stent and the vascular wall which are developed in a two-dimensional manner are displayed on the LCD monitor 113.

As is well known, the stent has a structure in which a thin metal wire is woven in a cylindrical shape. The structure is adopted so that the diameter of the stent is thin in a state until the stent is arranged inside the blood vessel and the diameter expands by an operation of a balloon or the like when the stent is positioned. Fig. 5 illustrates a simplified stent structure in order to facilitate the description. The stent generally has a structure including multiple cells 500 which are hollow portions configured to have a stent strut 501, and a structure in which the cells are connected to each other by a link 502. In any illustrated case, an example is illustrated in which a shape of one cell 500 has a diamond shape (square shape).

In addition, in the image of the vascular wall which is developed in the two-dimensional manner, the bifurcated portion of the blood vessel appears as a black region so that the brightness is extremely low. Therefore, a continuous region where the brightness has a predetermined value or smaller and a size thereof exceeds a predetermined size is extracted as the bifurcated portion of the blood vessel. The reference numeral 510 in Fig. 5 represents an example of the bifurcated portion of the blood vessel.

Furthermore, as illustrated in Fig. 5, a display 520 indicating a size of the guide wire is displayed on the LCD monitor 113. If a user selects a model number of the guide wire to be used from a guide wire list 521 which is a pull-down list, a circle 522 is displayed as a graphic having a size corresponding to the diameter of the selected guide wire. The size per pixel of the circle 522 displaying the size of the guide wire is equal to the size per pixel in the display of the two-dimensional vascular lumen image. Therefore, in order to select the cell through which the guide wire is to pass, the user can compare the size of the cell and the size of the guide wire with each other. In addition, a display menu 540 which is a pull-down menu for designating display content is prepared. Accordingly, the user can select desired display content from the display menu 540. Fig. 5 in which the two-dimensional vascular lumen image is displayed illustrates a state where "image display" is selected from the display menu.

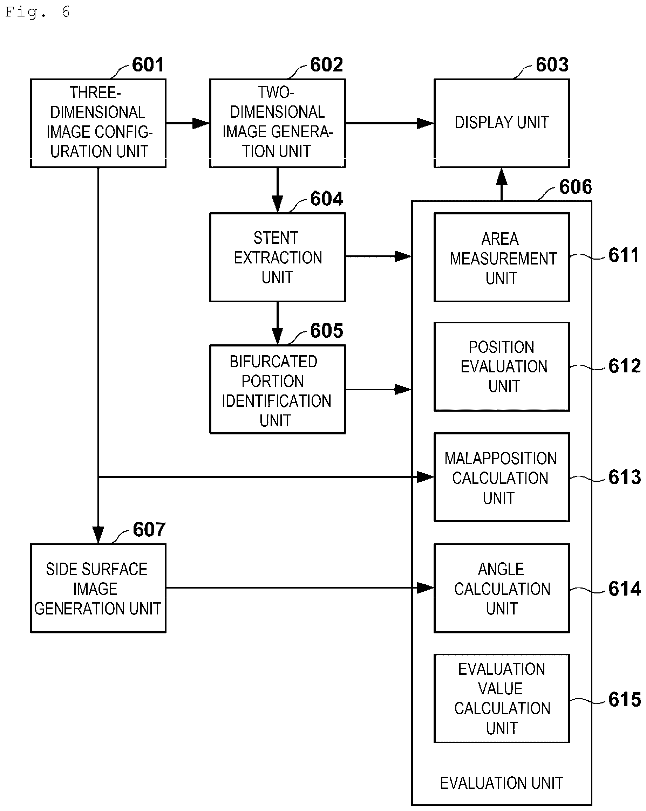

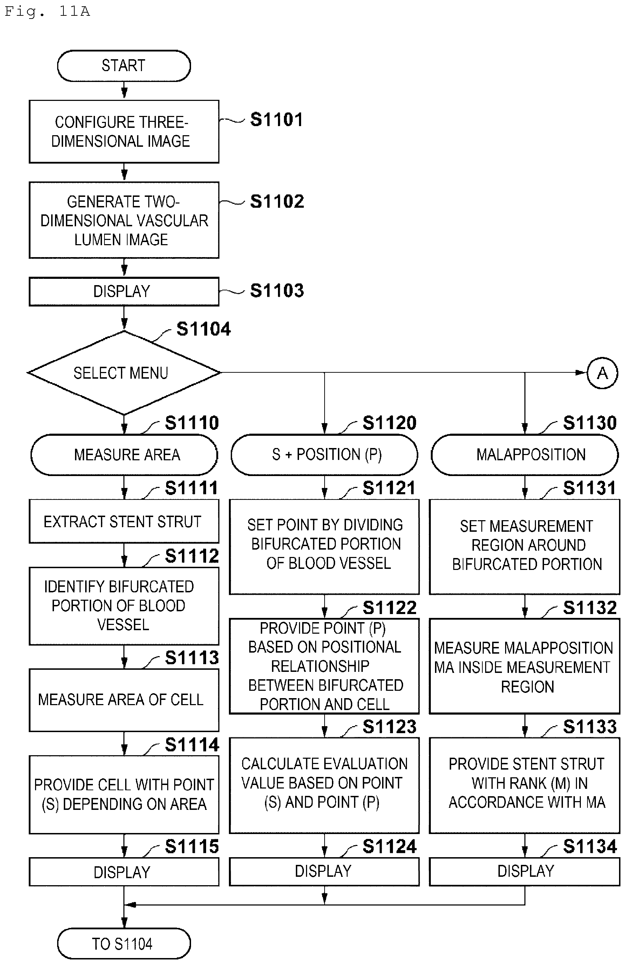

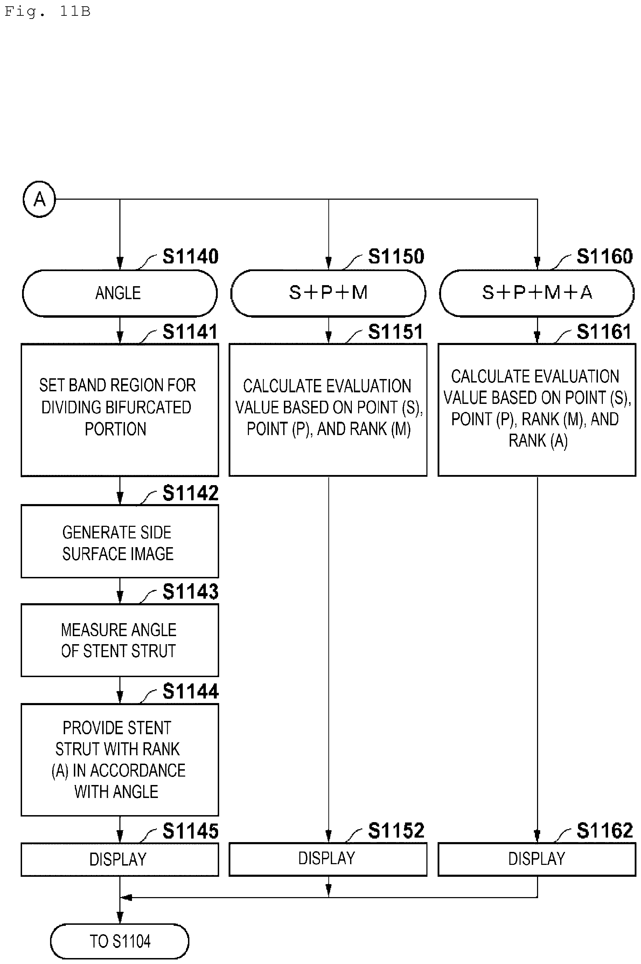

A support process of determining a guide wire route according to the imaging apparatus for diagnosis 100 configured as described above will be described. Fig. 6 is a block diagram illustrating a functional configuration example for performing the support process of determining the guide wire route. In addition, Figs. 11A and 11B are flowcharts for describing the determination support process according to the present embodiment. In the present embodiment, a CPU (not illustrated) inside the signal processing unit 201 executes a predetermined program so as to realize each functional block illustrated in Fig. 6 and to realize processes illustrated in Figs. 11A and 11B. However, the example is not limited thereto. For example, the functional block illustrated in Fig. 6 may be partially or entirely realized by using a dedicated processing IC.

As is described in Figs. 3A and 4, a three-dimensional image generation unit 601 generates a three-dimensional vascular image, based on interference light obtained by scanning of the imaging core 250 (S1101). A two-dimensional image generation unit 602 generates a two-dimensional vascular lumen image as illustrated in Fig. 5, based on the three-dimensional image generated by the three-dimensional image generation unit 601 (S1102). A display unit 603 displays the generated two-dimensional vascular lumen image and the display menu 540 on the LCD monitor 113 (S1103). If the "image display" is selected, the process returns to S1103.

A user can select desired display content from the display menu 540. If "area measurement result (S)" is selected from the display menu 540, the process proceeds from S1104 to S1110. An area measurement process of measuring an area of a division region divided by a stent strut extracted by a stent extraction unit is performed on the bifurcated portion of the blood vessel which is identified by a bifurcated portion identification unit. Through the above-described process, a stent extraction unit 604 extracts a stent strut 501 from a two-dimensional vascular lumen image (S1111). Through the above-described process, a bifurcated portion identification unit 605 extracts a bifurcated portion 510 of the blood vessel from the two-dimensional vascular lumen image (S1112).

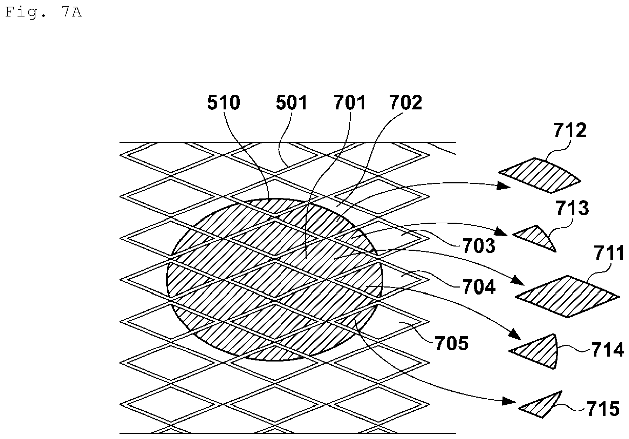

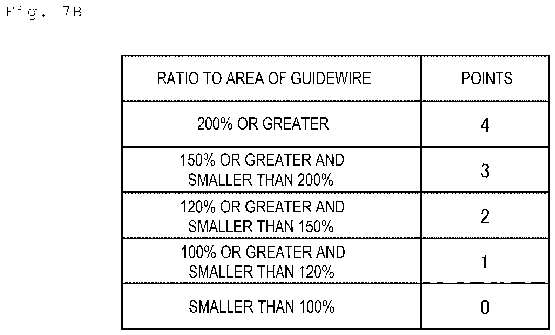

An evaluation unit 606 calculates an evaluation value for identifying a candidate of a cell through which a guide wire is to pass, based on the analysis of an overlapping state between each cell formed by the stent strut 501 and the bifurcated portion or a mounting state of the stent. An area measurement unit 611 of the evaluation unit 606 calculates an area of the division region divided by the stent strut 501 extracted by the stent extraction unit 604, in the bifurcated portion of the blood vessel which is identified by the bifurcated portion identification unit 605 (S1113). That is, in each cell formed by the stent strut, an area of the region overlapping the bifurcated portion identified by the bifurcated portion identification unit 605 is calculated. Figs. 7A and 7B are views for describing an operation of the area measurement unit 611. In Fig. 7A, in a case where the entire cell such as a region 701 is included in the bifurcated portion 510, a size of a region 711 of the entire cell is measured as the area. However, in regions 702 to 705, some regions protrude from the bifurcated portion 510. Thus, an area of regions which are respectively illustrated by the reference numerals 712 to 715 is measured.

If this area is larger, the guide wire is likely to pass through the area. Accordingly, it is possible to apply the area as one factor for identifying a candidate of the cell through which the guide wire is to pass. Fig. 7B illustrates an example where the area measurement unit 611 converts the area into a point of area measurement results. The area measurement unit 611 calculates a ratio between the area calculated in each cell and a cross-sectional area of the guide wire selected from the guide wire list 521, and provides a point (hereinafter, the point is referred to as a point S relating to the area) (S1114). For example, if an area of a portion overlapping the bifurcated portion of the cell is 1.3 times (130%) the area of the guide wire, the cell is provided with a point "2".

Then, the area measurement unit 611 changes a display form of the cell and displays the cell so that a user can identify the measured area, thereby providing an operator with support for determining a guide wire route (S1115). For example, the support includes displaying the cell colored in accordance with the above-described point in the display of the two-dimensional vascular lumen image in Fig. 5, or displaying the guide wire overlapping the above-described cell as an alternative manner.

The area measurement unit 611 is configured to measure a size (number of pixels) of a closed region formed by the stent strut in the bifurcated portion. However, a shape of the cell of the stent is generally complicated. In some cases, a portion of the cell has an open portion where no link is present. If a size of the closed region in the cell is measured, there is a possibility that a size of multiple cells connected to each other may be measured. However, the closed region including the multiple cells may be evaluated as one cell, or a constricted portion (portion narrower than a predetermined value) may be detected, divided, and evaluated.

Next, a case will be described where "evaluation result" → "S + position (P)" is selected from the display menu 540. In this case, the process proceeds from S1104 to S1120. The evaluation unit 606 calculates an evaluation value, based on the point S relating to the above-described area and a point P relating to a position to be described below. The evaluation unit 606 selects a candidate cell through which the guide wire is to pass in accordance with the evaluation value, and presents a user with the candidate cell.

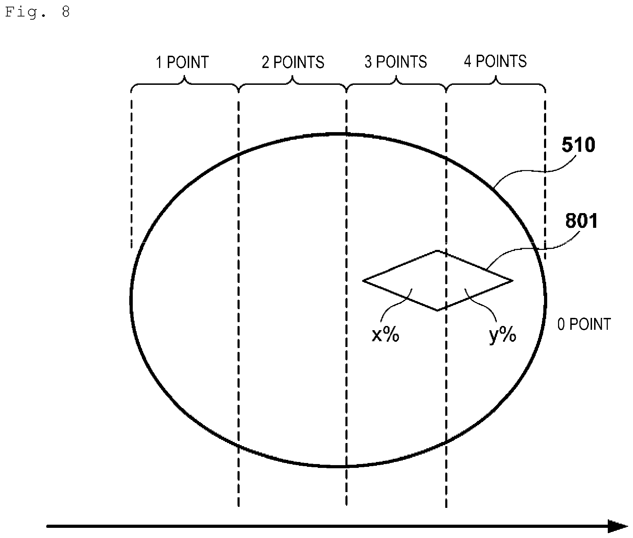

A position evaluation unit 612 evaluates a positional relationship between the position of the cell and the bifurcated portion, and provides the cell with the point P. In a case where the guide wire is caused to actually pass through the cell of the stent indwelling the bifurcated portion, the cell located at the farther position along the entering direction of the guide wire is preferentially selected. In the present embodiment, the bifurcated portion identified by the bifurcated portion identification unit 605 is divided into a predetermined number along the entering direction, and a point is set for each divided area (S1121). For example, as illustrated in Fig. 8, the bifurcated portion 510 is divided into four regions along the vascular axis so that the point becomes higher from the upstream side toward the downstream side. As a matter of course, the number of divided regions on the bifurcated portion 510 is not limited to four.

The position evaluation unit 612 determines the point relating to the position of each cell for each cell extracted from the two-dimensional vascular lumen image in accordance with the allocation of the point (S1122). For example, in a case where one cell crosses over a region of multiple points, it is assumed to use the point combined by an area ratio of each score region inside the cell. In a case of a cell 801 in Fig. 8, the point P relating to the position is calculated by using 3 × x (%) / 100 + 4 × y (%) / 100. The outside of the bifurcated portion 510 is set to "0 point".

An evaluation value calculation unit 615 calculates an evaluation value, based on the point S of each cell which is obtained in S1115 and the point P of each cell which is obtained in S1122 (S1123). The evaluation value calculation unit 615 displays a cell having a high evaluation value on the LCD monitor 113 so that the cell can be distinguished as a candidate cell (S1124). For example, in the display of the two-dimensional vascular lumen image illustrated in Fig. 5, the predetermined number of cells are colored and displayed in the order of higher evaluation values. In a case where the point S relating to the area is not calculated at this time, the processes in S1111 to S1114 may be performed so as to acquire the point S. In addition, alternatively, it is also possible to display a number indicating a rank on the cell in the order of higher evaluation values.

If "malapposition (MA)" is selected from the display menu 540, the process proceeds from S1104 to S1130, and an evaluation of the malapposition is performed.

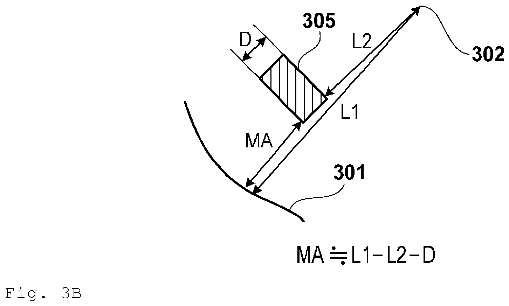

A malapposition calculation unit 613 calculates a distance between the stent strut and the vascular inner wall around the bifurcated portion. The malapposition is present in one of diagnosis-targeted items when a state where the stent is arranged inside the blood vessel is diagnosed. The malapposition means a gap between the stent and the vascular lumen surface, or a degree (distance) of the gap. In Fig. 3B, the reference numeral "MA" represents the malapposition. If in proximity to an edge of the stent, a distance from the rotation center 302 to the vascular lumen surface 301 outside the edge is set to L1, a distance from the rotation center 302 to an inner edge of the stent is set to L2, and a thickness of the stent is set to D, it is possible to easily understand from Fig. 3B that a relationship of L1 ≅ MA + D + L2 is satisfied. That is, the malapposition MA can be calculated as MA ≅ L1 - D -L2. The distances L1 and L2 among these can be directly obtained by scanning, and the thickness D of the stent is separately designated by a user. For example, the user inputs a type of the indwelling stent (for example, selects from a list), thereby providing the thickness D of the stent and calculating the malapposition MP.

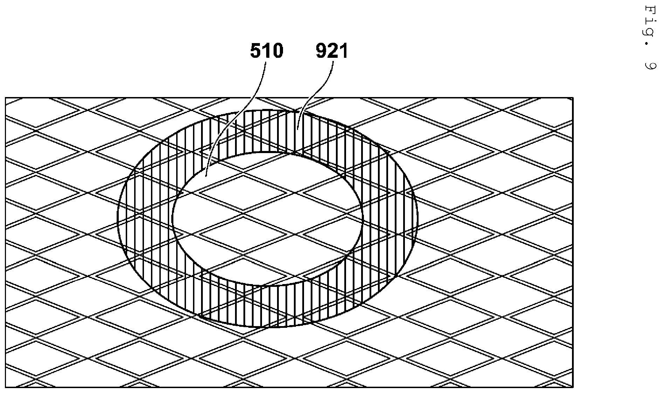

When the evaluation of the malapposition is performed, the malapposition calculation unit 613 first sets a calculation-targeted region of the malapposition (S1131). As illustrated in Fig. 9, the calculation-targeted region of the malapposition is set within a range 921 of a predetermined distance (for example, 5 mm) from the boundary of the bifurcated portion 510. The malapposition calculation unit 613 calculates the above-described malapposition (MA) with regard to each portion of the stent strut which is present within the range 921 (S1132), and ranks a size of the calculated malapposition (S1133). The rank allocated based on the malapposition is set to M. Then, the malapposition calculation unit 613 displays the rank M of the malapposition with regard to the stent strut within the range 921 so that the user can recognize the rank M (S1134). For example, in the display of the two-dimensional vascular lumen image in Fig. 5, the stent strut in which the rank M of the malapposition is calculated is displayed by being colored with a color corresponding to the calculated rank M. This display enables the user to easily recognize a tendency of the malapposition around the bifurcated portion 510, that is, a misaligned degree between the stent strut and the vascular wall. Accordingly, the user can refer to this display when selecting the cell through which the guide wire is to pass.

In a case where an "angle (A)" is selected from the display menu 540, the process proceeds from S1104 to S1140 so as to perform a process of calculating an angle of the stent strut with respect to the vascular wall.

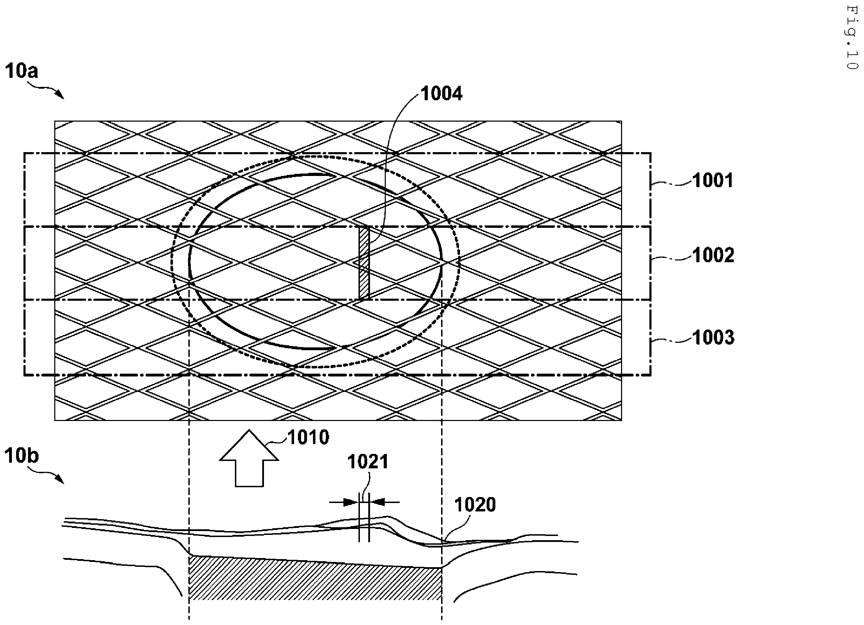

An angle calculation unit 614 calculates the angle of the stent strut with respect to the vascular wall. A side surface image obtained by observing the vascular wall and the stent in a side surface direction is applied when the angle is calculated. The side surface image is generated by a side surface image generation unit 607. As illustrated by 10a in Fig. 10, the side surface image generation unit 607 sets band-like regions (band regions 1001 to 1003) so that the bifurcated portion is divided into a predetermined number (three in the example of 10a in Fig. 10) in a direction orthogonal to the vascular direction (S1141). With regard to each band-like region, the side surface image generation unit 607 averages values of voxels within each region which are juxtaposed in an observation direction 1010 of a three-dimensional image, thereby obtaining the side surface image as illustrated by 10b in Fig. 10 for each region (S1142).

For example, in 10b of Fig. 10, multiple stent struts 501 juxtaposed in the observation direction 1010 in the band region 1002 are observed in a state of overlapping each other. Accordingly, in general, a stent strut image 1020 appears so as to be divided into multiple pieces or to become thick. Therefore, the angle calculation unit 614 bundles the stent strut image 1020 into one line, and calculates the angle with respect to a reference line obtained by performing first-order approximation on the stent strut around the bifurcated portion in each portion of the stent strut image 1020 (S1143). Furthermore, the angle calculation unit 614 ranks the calculated angle, and provides each portion of the stent strut with the rank (S1144).

The angle calculation unit 614 colors and displays the stent strut by using the rank relating to the angle obtained as described above (S1145). It is not necessary to calculate the angle for the entire range of the band regions 1001 to 1003. For example, it is sufficient to calculate the angle for a range 1005 in which the bifurcated portion 510 and the surroundings are provided with the range 921 illustrated in Fig. 9, out of the band regions 1001 to 1003. The side surface image as illustrated by 10b in Fig. 10 may be displayed on the LCD monitor 113.

In a case where "evaluation result" → "S + P + M" is selected from the display menu 540, the process proceeds from S1104 to S1150.

With regard to each cell, the evaluation value calculation unit 615 calculates an evaluation value serving as a guide wire route for each cell or for each stent strut, based on the point (S) relating to the area measured by the area measurement unit 611, the point (P) relating to the position acquired by the position evaluation unit 612, and the rank (M) of the malapposition calculated by the malapposition calculation unit 613 (S1151).

Then, the evaluation value calculation unit 615 displays the cell or the stent strut which has a high evaluation value calculated in S1151 on the LCD monitor 113 so that the cell or the stent strut can be distinguished as a candidate cell or a candidate stent strut (S1152). For example, in the display of the two-dimensional vascular lumen image in Fig. 5, the predetermined number of cells or stent struts are colored and displayed in the order of higher evaluation values.

In a case where "evaluation result" → "S + P + M + A" is selected from the display menu 540, the process proceeds from S1104 to S1160.

With regard to each cell, the evaluation value calculation unit 615 calculates an evaluation value serving as the guide wire route for each cell, based on the point (S) relating to the area measured by the area measurement unit 611, the point (P) relating to the position acquired by the position evaluation unit 612, the rank (M) of the malapposition provided by the malapposition calculation unit 613, and a rank (A) of the angle provided by the angle calculation unit 614 (S1161). The rank (A) of the angle is provided for the stent strut, similarly to the rank of the malapposition. Accordingly, a value obtained by statistically processing the rank of the angle provided for the stent strut configuring the cell is provided for the cell so as to be used.

Then, the evaluation value calculation unit 615 displays the cell which has a high evaluation value calculated in S1151 on the LCD monitor 113 so that the cell can be distinguished as a candidate cell (S1162). For example, in the display of the two-dimensional vascular lumen image illustrated in Fig. 5, the predetermined number of cells are colored and displayed in the order of higher evaluation values.

For example, the evaluation value calculation unit 615 adds the point (S) relating to the area to the point (P) relating to the position, thereby calculating an evaluation value for "S + position (P)". In addition, in a case where the stent strut configuring an evaluation-targeted cell includes the stent strut in which the rank (M) of the malapposition is calculated, an average value is calculated, based on the rank and the length of the stent strut provided with the rank. The value obtained in this way is set to be a point of the malapposition with respect to the cell. In addition, even in a case where the stent strut forming an evaluation-targeted cell includes the stent strut provided with the rank relating to the angle, an average value is similarly calculated, and the average value is set to be the point of the angle with respect to the cell.

The evaluation value calculation unit 615 calculates the evaluation value, based on the "point relating to the area", the "point relating to the position", the "point relating to the malapposition", and the "point relating to the angle", and displays the evaluation value as a candidate of the cell through which the guide wire is to pass, in the order of the calculated higher evaluation values. In a case where the "S + position (P)" of the "evaluation result" is selected from the display menu 540, the evaluation value calculation unit 615 calculates the evaluation value of each cell, based on the "point relating to the area" and the "point relating to the position". In addition, in a case where the "S + P + M" of the "evaluation result" is selected from the display menu 540, the evaluation value calculation unit 615 calculates the evaluation value of each cell, based on the "point relating to the area", the "point relating to the position", and the "point relating to the malapposition". In addition, in a case where the "S + P + M + A" of the "evaluation result" is selected from the display menu 540, the evaluation value calculation unit 615 calculates each evaluation value, based on the "point relating to the area", the "point relating to the position", the "point relating to the malapposition", and the "point relating to the angle".

The process according to the above-described embodiment may additionally have a menu and a process of calculating an evaluation value based on the area of the cell, the position of the cell, and the angle of the stent strut. That is, the "evaluation result" → "S + P + A" may be added to the display menu 540, and a process of calculating an evaluation value from the area (S), the position (P), and the angle (A) for each cell may be added.

As described above, according to the above-described embodiment, when the guide wire is caused to enter the laterally bifurcated portion via the cell of the stent indwelling the bifurcated portion of the blood vessel, it is possible to present a user with various reference indicators for selecting the cell through which the guide wire is to pass. For example, those are
▪ Arear overlapping the bifurcated portion of each cell,
▪ State of the malapposition of the stent strut, and
▪ Angle with respect to the vascular wall of the stent strut.

Furthermore, according to the above-described embodiment, based on the evaluation value calculated with reference to the above-described indicators, it is possible to present the user with a preferred cell as the cell through which the guide wire is to pass (access route for the guide wire). Accordingly, it is possible to effectively support the user to select the cell.

In the display of the two-dimensional vascular lumen image as illustrated in Fig. 5, in a case where multiple bifurcated portions are identified, the user is enabled to designate a desired bifurcated portion. In addition, in the display of the two-dimensional vascular lumen image, in a case where the bifurcated portion which allows the guide wire to enter is vertically divided, the position illustrated by line A-A in Fig. 4 is changed, and the two-dimensional vascular lumen image is regenerated so that the target bifurcated portion is continuous.

As is understood from the above-described embodiment, a part of the characteristics according to the embodiment is performed by the signal processing unit 201 configured to include at least a microprocessor. A program causes the microprocessor to execute the processes in order to realize the function. Therefore, as a matter of course, the program is also included in the scope of the present invention. In addition, the program is normally stored in a computer-readable recording medium such as CD-ROM, DVD-ROM, and the like. The program can cause the microprocessor to execute the processes by the program being set in a reading device (CD-ROM drive or the like) belonging to a computer and being copied to or installed in a system. Therefore, it is apparent that the related computer-readable recording medium is also included in the scope of the present invention.

## Claims

1. An imaging apparatus for diagnosis (100) which reconfigures a cross-sectional image of a blood vessel by having a wavelength swept light source (203) emitting light toward a lumen surface of the blood vessel and having a photodiode (204) detecting interference light between the reflected light and reference light, comprising:
identification means for identifying a bifurcated portion (510) of the blood vessel from the cross-sectional image;
extraction means for extracting a stent strut (501) from the cross-sectional image; **characterized by**
area measurement means configured to measure an area of a division region (711, 712, 713, 714, 715) in the cross-sectional image, in which the bifurcated portion (510) identified by the identification means is divided into regions (701, 702, 703, 704, 705) by the stent strut (501) extracted by the extraction means;
display means configured to display the cross-sectional image and information of the area (711, 712, 713, 714, 715) measured by the area measurement means; and
wherein the display means is further configured to display a graphic indicating a size of a guide wire.

2. The imaging apparatus for diagnosis according to Claim 1,
wherein the identification means identifies a continuous region, in the cross-sectional image, whose brightness is equal to or smaller than a predetermined value and whose size exceeds a predetermined size, as the bifurcated portion (510).

3. The imaging apparatus for diagnosis according to any one of Claims 1 to 2, further comprising:
evaluation means for calculating an evaluation value with regard to a cell (500) formed by the stent strut (501), based on the area (711, 712, 713, 714, 715) measured by the area measurement means and a position of the cell (500) relative to the bifurcated portion (510),
wherein the display means changes a display form of the cell (500) based on the evaluation value calculated by the evaluation means and displays the cell.

4. The imaging apparatus for diagnosis according to any one of Claims 1 to 2, further comprising:
distance calculation means for calculating a distance between the lumen surface (301) of the blood vessel and the stent strut (501), based on data obtained from the interference light,
wherein the display means changes a display form of the stent strut (501), based on the distance calculated by the distance calculation means.

5. The imaging apparatus for diagnosis according to Claim 4, further comprising:
evaluation means for calculating an evaluation value with regard to a cell (500) formed by the stent strut (501), based on the area (711, 712, 713, 714, 715) measured by the area measurement means, a position of the cell relative to the bifurcated portion (510), and the distance of the stent strut (501) configuring the cell (500), which is calculated by the distance calculation means,
wherein the display means changes a display form of the cell based on the evaluation value calculated by the evaluation means and displays the cell (500).

6. The imaging apparatus for diagnosis according to any one of Claims 1 to 2, further comprising:
side surface image generation means for generating a side surface image along an axial direction of the blood vessel, with regard to a region of the bifurcated portion (510) of the blood vessel which is identified by the identification means and surroundings thereof; and
angle calculation means for calculating an angle (A) of the stent strut in the axial direction, based on the side surface image,
wherein the display form of the stent strut is changed based on the angle (A) calculated by the angle calculation means.

7. The imaging apparatus for diagnosis according to Claim 6, further comprising:
evaluation means for calculating an evaluation value with regard to a cell (500) formed by the stent strut (501), based on the area (711, 712, 713, 714, 715) measured by the area measurement means, a position of the cell (500) relative to the bifurcated portion (510), and the angle (A) calculated by the angle calculation means of the stent strut (501) configuring the cell,
wherein the display means changes a display form of the cell (500) based on the evaluation value calculated by the evaluation means and displays the cell.

8. A computer program for determining an access route for a guide wire by reconfiguring a cross-sectional image of a blood vessel, comprising instructions to cause an imaging apparatus for diagnosis, which comprises a wavelength swept light source (203) for emitting light toward a lumen surface of the blood vessel and a photodiode (204) for detecting interference light between the reflected light and reference light, to execute the steps of:
an obtaining step of obtaining the cross-sectional image from the detected interference light;
an identification step of identifying a bifurcated portion (510) of the blood vessel from the cross-sectional image;
an extracting step of extracting a stent strut (501) from the cross-sectional image; **characterized by**
an area measurement step of measuring an area of a division region (711, 712, 713, 714, 715) in the cross-sectional image, in which the bifurcated portion identified in the identification step is divided into regions (701, 702, 703, 704, 705) by the stent strut (501) extracted in the extraction step; and
a display step of causing display means to display the cross-sectional image and information of the area (711, 712, 713, 714, 715) measured in the area measurement step; wherein the display step is further causing the display means to display a graphic indicating a size of a guide wire.

9. A computer-readable storage medium storing the program according to Claim 8.

## Patentansprüche

1. Ein Bildgebungsgerät zur Diagnose (100), das eine Querschnittsaufnahme eines Blutgefäßes über eine wellenlängenvariable Lichtquelle (203) umkonfiguriert, die Licht an eine Lumenoberfläche des Blutgefäßes ausgibt und eine Fotodiode (204) aufweist, die Störlicht zwischen dem reflektierten Licht und dem Referenzlicht erfasst, mit:
einem Erfassungsmittel zur Identifizierung eines gegabelten Abschnitts (510) des Blutgefäßes in der Querschnittsaufnahme,
einem Auszugsmittel zur Extraktion eines Stentstegs (501) aus der Querschnittsaufnahme,
**dadurch gekennzeichnet, dass**
ein Flächenmessmittel zur Messung einer Fläche eines Unterteilungsbereichs (711, 712, 713, 714, 715) in der Querschnittsaufnahme vorhanden ist, in der der vom Erfassungsmittel identifizierte gegabelte Abschnitt (510) durch den vom Auszugsmittel extrahierten Stentsteg (501) in Regionen (701, 702, 703, 704, 705) unterteilt wird,
ein Anzeigemittel vorhanden ist, das zur Anzeige der Querschnittsaufnahme und von Informationen zum Bereich (711, 712, 713, 714, 715) ausgelegt ist, der vom Messmittel gemessen wurde, und
wobei das Anzeigemittel weiterhin zur Anzeige einer Grafik ausgelegt ist, die die Größe eines Führungsdrahts vorgibt.

2. Das Bildgebungsgerät zur Diagnose gemäß Anspruch 1,
wobei das Erfassungsgerät eine durchgehende Region in der Querschnittsaufnahme als gegabelten Abschnitt (510) identifiziert, dessen Helligkeit kleiner gleich einem vorbestimmten Wert ist und dessen Größe einen vorbestimmten Wert übersteigt.

3. Das Bildgebungsgerät zur Diagnose gemäß einem der Ansprüche 1 bis 2, das weiterhin Folgendes aufweist:
ein Auswertungsmittel zur Berechnung eines Evaluationswerts in Bezug auf eine Zelle (500), die vom Stentsteg (501) gebildet wird, auf Grundlage der vom Flächenmessmittel gemessenen Fläche (711, 712, 713, 714, 715) und einer Position der Zelle (500) in Bezug auf den gegabelten Abschnitt (510),
wobei das Anzeigemittel die Anzeigeform der Zelle (500) auf Grundlage des durch das Auswertungsmittel berechneten Werts ändert und die Zelle anzeigt.

4. Das Bildgebungsgerät zur Diagnose gemäß einem der Ansprüche 1 bis 2, das weiterhin Folgendes aufweist:
Entfernungsmessmittel zur Berechnung einer Distanz zwischen der Lumenoberfläche (301) des Blutgefäßes und dem Stentsteg (501) auf Grundlage von aus dem Störlicht erhaltenen Daten,
wobei das Anzeigemittel die Anzeigeform des Stentstegs (501) auf Grundlage des durch das Entfernungsmessmittel berechneten Distanz ändert.

5. Das Bildgebungsgerät zur Diagnose gemäß Anspruch 4, das weiterhin Folgendes aufweist:
Auswertungsmittel zur Berechnung eines Evaluationswerts in Bezug auf eine Zelle (500), die vom Stentsteg (501) gebildet wird, auf Grundlage der vom Flächenmessmittel gemessenen Fläche (711, 712, 713, 714, 715), einer Position der Zelle in Bezug auf den gegabelten Abschnitt (510) und der die Zelle (500) konfigurierenden Distanz des Stentstegs (501), die vom Entfernungsmessmittel berechnet wird,
wobei das Anzeigemittel die Anzeigeform der Zelle auf Grundlage des durch das Auswertungsmittel berechneten Werts ändert und die Zelle (500) anzeigt.

6. Das Bildgebungsgerät zur Diagnose gemäß einem der Ansprüche 1 bis 2, das weiterhin Folgendes aufweist:
Seitenflächen-Bildgenerierungsmittel zur Generierung eines Seitenflächenbilds entlang einer axialen Richtung des Blutgefäßes in Bezug auf eine Region des gegabelten Abschnitts (510) des Blutgefäßes, die durch Erfassungsmittel identifiziert wird, und deren Umgebung, und
Winkelberechnungsmittel zur Berechnung eines Winkels (A) des Stentstegs in axialer Richtung auf Grundlage des Seitenflächenbilds,
wobei die Anzeigeform des Stentstegs (501) auf Grundlage des durch das Winkelberechnungsmittel berechneten Winkels (A) geändert wird.

7. Das Bildgebungsgerät zur Diagnose gemäß Anspruch 6, das weiterhin Folgendes aufweist:
Auswertungsmittel zur Berechnung eines Evaluationswerts in Bezug auf eine Zelle (500), die vom Stentsteg (501) gebildet wird, auf Grundlage der vom Flächenmessmittel gemessenen Fläche (711, 712, 713, 714, 715), einer Position der Zelle (500) in Bezug auf den gegabelten Abschnitt (510) und den durch das Winkelberechnungsmittel des Stentstegs (501) berechneten, die Zelle konfigurierenden Winkel (A),
wobei das Anzeigemittel die Anzeigeform der Zelle (500) auf Grundlage des durch das Auswertungsmittel berechneten Werts ändert und die Zelle anzeigt.

8. Ein Computerprogramm zur Bestimmung eines Zugangspfads für einen Führungsdraht durch Umkonfigurieren einer Querschnittsaufnahme eines Blutgefäßes mit Anweisungen, die ein Bildgebungsgerät zur Diagnose mit einer wellenlängenvariablen Lichtquelle (203) zur Emission von Licht an eine Lumenoberfläche und eine Fotodiode (204) zur Erfassung von Störlicht zwischen dem reflektierten Licht und dem Referenzlicht veranlasst, und das folgende Schritte ausführt:
einen Aufnahmeschritt zur Aufnahme einer Querschnittsaufnahme über das erfasste Störlicht,
einen Erfassungsschritt zur Identifizierung eines gegabelten Abschnitts (510) des Blutgefäßes in der Querschnittsaufnahme,
einen Auszugsschritt zur Extraktion eines Stentstegs (501) aus der Querschnittsaufnahme,
**dadurch gekennzeichnet, dass**
ein Flächenmessschritt zur Messung einer Fläche einem Unterteilungsbereich (711, 712, 713, 714, 715) in der Querschnittsaufnahme vorhanden ist, in dem der vom Erfassungsschritt identifizierte gegabelte Abschnitt durch den vom Auszugsschritt extrahierten Stentsteg (501) in Regionen (701, 702, 703, 704, 705) unterteilt wird, und
ein Anzeigeschritt vorhanden ist, der das Anzeigemittel zur Anzeige der Querschnittsaufnahme und von Informationen zur im Messschritt gemessenen Fläche (711, 712, 713, 714, 715) veranlasst,
wobei der Anzeigeschritt weiterhin zur Anzeige einer Grafik führt, die die Größe eines Führungsdrahts vorgibt.

9. Ein computerlesbares Speichermedium zur Speicherung des Programms gemäß Anspruch 8.

## Revendications

1. Appareil d'imagerie de diagnostic (100) lequel reconfigure une image de coupe transversale d'un vaisseau sanguin en ayant une source de lumière à balayage de longueur d'onde (203) émettant une lumière vers une surface de lumière du vaisseau sanguin et présentant une photodiode (204) détectant une lumière d'interférence entre la lumière réfléchie et une lumière de référence, comprenant :
un moyen d'identification destiné à identifier une partie de bifurcation (510) du vaisseau sanguin à partir de l'image de coupe transversale ;
un moyen d'extraction destiné à extraire une entretoise de stent (501) à partir de l'image de coupe transversale ;
**caractérisé par**
un moyen de mesure de superficie configuré pour mesurer une superficie d'une zone de division (711, 712, 713, 714, 715) sur l'image de coupe transversale, dans laquelle la partie de bifurcation (510) identifiée par le moyen d'identification est divisée en zones (701, 702, 703, 704, 705) par l'entretoise de stent (501) extraite par le moyen d'extraction ;
un moyen d'affichage configuré pour afficher l'image de coupe transversale et l'information de la superficie (711, 712, 713, 714, 715) mesurée par le moyen de mesure de superficie ; et où le moyen d'affichage est en outre configuré pour afficher un graphique indiquant une dimension d'un fil guide.

2. Appareil d'imagerie de diagnostic selon la revendication 1,
dans lequel le moyen d'identification identifie une zone continue, sur l'image de coupe transversale, dont la luminosité est inférieure ou égale à une valeur prédéterminée et dont la dimension dépasse une dimension prédéterminée, comme la partie de bifurcation (510).

3. Appareil d'imagerie de diagnostic selon l'une quelconque des revendications 1 et 2, comprenant en outre :
un moyen d'évaluation destiné à calculer une valeur d'évaluation concernant une cellule (500) formée par l'entretoise de stent (501), sur la base de la superficie (711, 712, 713, 714, 715) mesurée par le moyen de mesure de superficie et d'une position de la cellule (500) par rapport à la partie de bifurcation (510),
où le moyen d'affichage modifie une forme d'affichage de la cellule (500) sur la base de la valeur d'évaluation calculée par le moyen d'évaluation et affiche la cellule.

4. Appareil d'imagerie de diagnostic selon l'une quelconque des revendications 1 et 2, comprenant en outre :
un moyen de calcul de distance destiné à calculer une distance entre la surface de lumière (301) du vaisseau sanguin et l'entretoise de stent (501), sur la base de données obtenues à partir de la lumière d'interférence,
où le moyen d'affichage modifie une forme d'affichage de l'entretoise de stent (501), sur la base de la distance calculée par le moyen de calcul de distance.

5. Appareil d'imagerie de diagnostic selon la revendication 4, comprenant en outre :
un moyen d'évaluation destiné à calculer une valeur d'évaluation concernant une cellule (500) formée par l'entretoise de stent (501), sur la base de la superficie (711, 712, 713, 714, 715) mesurée par le moyen de mesure de superficie, d'une position de la cellule par rapport à la partie de bifurcation (510), et de la distance de l'entretoise de stent (501) configurant la cellule (500), laquelle est calculée par le moyen de calcul de distance,
où le moyen d'affichage modifie une forme d'affichage de la cellule sur la base de la valeur d'évaluation calculée par le moyen d'évaluation et affiche la cellule (500).

6. Appareil d'imagerie de diagnostic selon l'une quelconque des revendications 1 et 2, comprenant en outre :
un moyen de génération d'image de surface latérale destiné à générer une image de surface latérale le long d'une direction axiale du vaisseau sanguin, concernant une zone de la partie de bifurcation (510) du vaisseau sanguin laquelle est identifiée par le moyen d'identification et les environs de celle-ci ; et
un moyen de calcul d'angle destiné à calculer un angle (A) de l'entretoise de stent dans la direction axiale, sur la base de l'image de surface latérale,
où la forme d'affichage de l'entretoise de stent est modifiée sur la base de l'angle (A) calculé par le moyen de calcul d'angle.

7. Appareil d'imagerie de diagnostic selon la revendication 6, comprenant en outre :
un moyen d'évaluation destiné à calculer une valeur d'évaluation concernant une cellule (500) formée par l'entretoise de stent (501), sur la base de la superficie (711, 712, 713, 714, 715) mesurée par le moyen de mesure de superficie, d'une position de la cellule (500) par rapport à la partie de bifurcation (510), et de l'angle (A) calculé par le moyen de calcul d'angle de l'entretoise de stent (501) configurant la cellule,
où le moyen d'affichage modifie une forme d'affichage de la cellule (500) sur la base de la valeur d'évaluation calculée par le moyen d'évaluation et affiche la cellule.

8. Programme informatique destiné à déterminer une voie d'accès pour un fil guide en reconfigurant une image de coupe transversale d'un vaisseau sanguin, comprenant des instructions pour amener un appareil d'imagerie de diagnostic, lequel comprend une source de lumière à balayage de longueur d'onde (203) destinée à émettre une lumière vers une surface de lumière du vaisseau sanguin et une photodiode (204) destinée à détecter une lumière d'interférence entre la lumière réfléchie et une lumière de référence, à exécuter les étapes suivantes :
une étape d'obtention consistant à obtenir l'image de coupe transversale à partir de la lumière d'interférence détectée ;
une étape d'identification consistant à identifier une partie de bifurcation (510) du vaisseau sanguin à partir de l'image de coupe transversale ;
une étape d'extraction destinée à extraire une entretoise de stent (501) à partir de l'image de coupe transversale ;
**caractérisé par**
une étape de mesure de superficie destinée à mesurer une superficie d'une zone de division (711, 712, 713, 714, 715) sur l'image de coupe transversale, dans laquelle la partie de bifurcation identifiée lors de l'étape d'identification est divisée en zones (701, 702, 703, 704, 705) par l'entretoise de stent (501) extraite lors de l'étape d'extraction ; et
une étape d'affichage destinée à amener un moyen d'affichage à afficher l'image de coupe transversale et l'information de la superficie (711, 712, 713, 714, 715) mesurée lors de l'étape de mesure de superficie ; où l'étape d'affichage amène en outre le moyen d'affichage à afficher un graphique indiquant une dimension d'un fil guide.

9. Support de stockage lisible par ordinateur stockant le programme selon la revendication 8.
